(19) Europäisches Patentamt
European Patent Office
Office européen des brevets

(11) Publication number: 0 224 201 B1

(12) **EUROPEAN PATENT SPECIFICATION**

(45) Date of publication of patent specification: **11.09.91**

(51) Int. Cl.[5]: **C08G 65/32**

(21) Application number: **86116085.1**

(22) Date of filing: **20.11.86**

(54) **Functionalized perfluoropolyethers and process for their preparation.**

(30) Priority: **20.11.85 IT 2292085**

(43) Date of publication of application:
**03.06.87 Bulletin 87/23**

(45) Publication of the grant of the patent:
**11.09.91 Bulletin 91/37**

(84) Designated Contracting States:
**AT BE CH DE ES FR GB IT LI NL SE**

(56) References cited:
**EP-A- 0 167 258**
**BE-A- 764 110**
**DE-A- 1 816 752**
**GB-A- 1 192 238**
**US-A- 4 523 039**

(73) Proprietor: **AUSIMONT S.p.A.**
**31, Foro Buonaparte**
**I-20121 Milano(IT)**

(72) Inventor: **Caporiccio, Gerardo**
**13, via E. Filiberto**
**I-20149 Milan(IT)**
Inventor: **Viola, Gian Tommaso**
**13, via Pignocchi**
**I-48015 Cervia(IT)**
Inventor: **Marchionni, Giuseppe**
**8, via Vallisneri**
**I-20133 Milan(IT)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40(DE)**

**EP 0 224 201 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the preparation of low molecular weight perfluoropolyethers by a process for the cracking of high molecular weight perfluoropolyethers obtained from the photooxidation of perfluoroolefins or by polymerization with opening of the ring of partially fluorinated oxetane compounds or by fluorination of hydrogenated polyalkylene oxides.

It is generally known that the methods employed for the preparation of the above-mentioned perfluoropolyethers lead to perfluoropolyethers having, for the most part, a molecular weight that is too high. These high molecular weight perfluoropolyethers have only limited practical applications. It is well-known that applications in the field of electronics require very low average molecular weight perfluoroethers while medium molecular weight perfluoropolyethers are employed as operative fluids for high-vacuum pumps.

GB-A-1,192,238 discloses a process for the modification of certain perfluoropolyethers, i.e., the (fluoroformate) end groups thereof, in the optional presence of, e.g., compounds of Zn, Cd, Al or Fe. Said compounds are preferably employed in stoichiometric amounts.

BE-A-764,110 relates to the production of cyclic perfluoropolyethers by subjecting certain linear perfluoropolyethers to temperatures between 150 °C and 550 °C. Optionally said conversion may be conducted in the presence of a catalyst, e.g., an aluminium compound.

An object of the present invention is to provide a process for reducing the mean molecular weight of the above high molecular weight perfluoropolyethers by cracking of the perfluoropolyether chains until the desired value is achieved.

Accordingly, the present invention provides a process for the cracking of perfluoropolyethers of the following classes:

I) $R_fO(CF_2CF_2O)_nR_f$

II) $A(CF_2CF_2CF_2O)_nB$

III) $E(C_2F_4O)_m(C_3F_6O)_p(CF_2O)_q(\underset{\displaystyle CF_3}{\underset{|}{C}}FO)_rD$

wherein n, m, p, q and r are integers, n ranges from 2 to 200; m, p, q and r range from 1 to 100 and the sum m+p+q+r ranges from 4 to 400; $R_f$ is $CF_3$ or $C_2F_5$; A is F or $OR_f$; B and D are perfluoroalkyl groups having 1 to 3 carbon atoms; and E is F, or $OR'_f$, wherein $R'_f$ is a perfluoroalkyl group having 1 to 3 carbon atoms.

This cracking process comprises heating perfluoropolyethers of formulae (I), (II) or (III) to temperatures of from 150 to 380 °C in the presence of a catalyst composed of fluorides, oxyfluorides or oxides of transition metals selected from Ti, V, Cr, Mn, Fe, Co, Ni, Cu, Zr, Mo, Zn or of Al, Sn and Sb.

The amount of catalyst utilized varies from 0.1 to 10% by weight, based on the weight of the starting perfluoropolyether. Due to the disclosure of EP-A-167258, a document relevant under Art, 54(3) EPC, for the designated states BE, DE, FR, GB, NL and SE there is the proviso that in the case of perfluoropolyethers of formula II the catalyst does not comprise fluorides and/or oxyfluorides of Ti, V, Co, Ni or Al or mixtures thereof in accounts of from 0.1 to 2 percent by weight.

The process of the invention may also be used for the perfluoropolyethers of class (III) which are directly obtained from the photooxidation of the mixture of perfluoroolefins, wherein a part of the end groups are acid groups -COF. In the latter case, greater amounts of catalyst as well as longer treatment times and higher temperatures are employed.

Analogously, the products of class (II), directly obtained from the oxetane ring opening step as indicated above, may be utilized as fluoropolyethers to be subjected to cracking. In this case, the monomeric units are $-CH_2CF_2CF_2O-$. This polyether is described in EP-A-148 482.

The reaction times can vary greatly. They may, for example, be of the order of from 1 minute to a few hours, preferaby, from 3 minutes to 5 hours. Therefore, by properly selecting the reaction conditions and the characteristics of the catalyst utilized, it is possible to obtain products dominantly having a predetermined mean molecular weight, starting from high molecular weight perfluoropolyethers.

The starting perfluoropolyethers of class (I) may, for example, be prepared according to US-A-4 534 039, the products of class (II) according to EP-A-148 482 and the products of class (III) according to US-A-3

665 041.

As already mentioned, the advantage offered by the present process resides in the fact that it is possible to modify the molecular weight distribution of the product resulting from a usual perfluoropolyether preparation method by enriching it, in particular, with the most useful fractions. Therefore, an important degree of flexibility is imparted to the production process in order to obtain products having a predetermined molecular weight on which, as is known, the viscosity and the vapour tension of the final products depend.

The crucial conditions for achieving this object are:

a) Maintaining the temperature in the range of from 150 to 380 °C, as a function of the type and amount of catalyst;

b) The concentration and type of catalyst employed.

Catalysts which are suitable for the process according to the invention can be obtained starting from compounds of the above-mentioned elements and also compounds other than fluorides or oxyfluorides provided they are capable, under reaction conditions, of being converted at least partially to the corresponding fluorides or oxyfluorides due to the release of fluorine from the perfluoropolyether product to be treated.

In the case of cobalt and nickel it has been ascertained that effective catalysts are the fluorides of the metals in their highest oxidation state. Good results can also be obtained by using different halides of Co and/or Ni in an oxidation state lower than the maximum one, provided a gaseous fluorine stream is introduced into the reaction vessel, such stream causing the in situ formation of the corresponding fluoride in the highest oxidation state.

Titanium may suitably be employed as $TiOF_2$.

According to a preferred method, this compound is prepared in situ by heating the perfluoropolyether to temperatures above 300 °C in an oxygen stream and in the presence of titanium or alloys thereof.

Among the cited oxides $TiO_2$ and $Al_2O_3$ are particularly suited for the process of the invention.

A catalyst particularly suitable for the process according to the invention is aluminium fluoride having specific morphological and structural characteristics. This catalyst is described in EP-A-167 258.

The catalyst, consisting of $AlF_3$, can also be formed in situ during the reaction by adding anhydrous $AlBr_3$ to the starting perfluoropolyether. Under the reaction conditions, in fact, a substitution of bromine by the fluorine of the perfluoropolyether can take place.

The fluorides and the oxyfluorides may also be prepared in situ starting from the corresponding halides, operating in the presence of fluorine.

In the cracking process, a decrease in the molecular weight (MW) of the perfluoropolyethers and an increase in acidity take place. The rupture of the ethereal bond gives rise to the formation of an acid end group of the type: $-(O)-CF_2COF$ and $-(O)CF_2CF_2COF$ for classes (I) and (II), $-(O)-CF_2COF$,

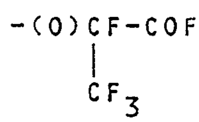

or a keto group

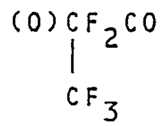

for class (III) and a neutral end group, namely a perfluoroalkyl group having 1 to 3 carbon atoms. Oxygen is present in the acid end groups if the end group is not already bound to an oxygen of the chain.

In all cases the resulting product has a molecular weight lower than that of the starting product and a residual acidity that, if it is of interest to obtain neutral PFPE, can be eliminated by treatment with elemental fluorine in the presence of UV radiation, as described in EP-A-193 028 or, in the absence of UV radiation; at temperatures ranging from 120 to 250 °C, thus obtaining a completely neutral perfluoropolyether having a lower mean molecular weight

Thus, the present invention provides a method for the high-yield conversion of high molecular weight perfluoropolyethers to neutral perfluoropolyethers having a lower molecular weight which - as explained above - are particularly useful.

Another object of the invention resides in the possibility of obtaining, by the process of the invention, new functionalized perfluoropolyethers having general formulae corresponding to the ones of classes (I) and (II), wherein both end groups are of the type:

1) $-OCF_2COF$ in class (I), and

2) $-OCF_2CF_2COF$ in class (II).

As previously mentioned, neutral and acid end groups are obtained from the cracking process illustrated above.

The neutral end groups are of the type:

3) $-OCF_2CF_3$

4) $-OCF_2CF_2CF_3$.

If the reaction is conducted in the absence of $F_2$, the number of acid end groups can be maximized by reducing the time of contact with the catalyst; conversely, if the reaction is conducted in the presence of $F_2$, the acid end groups are almost fully eliminated as a result of the known neutralization reaction induced by fluorine.

Due to the linearity of the repeating monomeric units, molecules are formed which have at their ends neutral (i.e., perfluoroalkyl) and acid end groups in a substantially unitary ratio but statistically distributed at the ends of the chains. Therefore, molecules with two neutral end groups of types 3) and 4), molecules with a neutral end group and an acid end group and molecules with two acid end groups of types 1) and 2) are present. If the degradation reaction is not conducted in a proper manner as already mentioned, i.e., minimizing the contact time of the perfluoropolyether with the catalyst and, under continuous separation from the reaction mixture of the low molecular weight products resulting from the reaction, for example, by distillation, optionally at reduced pressure, the amount of neutral molecules will increase due to the decarbonylation of end groups 1) and 2). The subsequent separation of the acid molecules from the neutral molecules is carried out by salification with alkali and subsequent distillation, optionally in a vapour stream.

The residue consists of the alkaline salts of the monoand difunctional acids which, by acidification with strong acids, provide the acid perfluoropolyethers with end groups -COOH.

The perfluoropolyethers with acid end groups thus prepared can be converted to perfluoropolyethers having other functional groups according to known reactions, for example, by conversion of the carboxylic group to amide and ester groups which, in turn, can be converted to other functional groups such as, for example, nitrile, isocyanate, hydroxyl, etc.

Methods of preparing several functional groups are described in US-A-4 094 911.

The functionalized perfluoropolyethers can be utilized as monomers to obtain polymers in polyaddition or polycondensation reactions which have low glass transition temperatures (Tg) $< -78^\circ C$.

Polymers obtainable are, for example, of the polyester, polyalcohol, polyurethane, polyether, polyamide, polyamine or polyacrylate type.

In a particularly advantageous embodiment of the present invention the dissociation process is carried out under such working conditions that the products having a sufficiently low molecular weight are continuously separated from the reaction mixture. This result is achieved by combining the chemical dissociation treatment with a fractionation treatment, for example, by means of distillation or flash separation or molecular distillation of the dissociation product, such treatment being carried out immediately after the cracking or simultaneously therewith.

The following examples illustrate the invention but do not limit it in any way.

EXAMPLE 1

10 g of a perfluoropolyether of the structure $CF_3(OCF_2CF_2)_5OC_2F_5$, prepared according to example 1 of US-A-4 523 039, and 0.1 g of $\gamma$-$AlF_3$, prepared according to example 1 of Italian patent no. 1176179 were fed to a Hastelloy® auto-clave having a volume of 20ml and being heated by means of an oil bath. This mixture was heated to a temperature of 240°C for 10 minutes. The resulting product was then evaporated under vacuum and collected in a trap cooled to -80°C with dry ice/acetone. The product weighed 9 g and was composed of a mixture of acid and neutral molecules in a ratio of 20:80. An analysis of the mixutre revealed that it consisted of molecules $A(OCF_2\ CF_2)_nOB$, wherein A is the same or different from B and represents $-CF_3$, $-CF_2\ CF_3$ or $-CF_2COF$, with n ranging from 0 to 3.

EXAMPLE 2

Using the apparatus and the starting perfluoropolyethers of example 1 but 0.1 g of $TiO_2$, the reaction was conducted at 220°C for 10 minutes, whereafter 9.3 g of a low-boiling product was obtained.

The molecular structure was analogous to that of the product of example 1, the only exception being a different distribution of acid and neutral molecules, the ratio being 3:10.

EXAMPLE 3

1000 g of a PFPE having the structure $F(CF_2CF_2CF_2O)_{23}CF_2CF_3$, prepared according to the procedure of example 14 of EP-A-148 482 and having an average molecular weight of 4000 a.m.u., as well, as 10 g of $AlF_3$ of type 1), prepared according to Italian patent no.1176179, were fed to a metal reactor having a volume of 1200 ml and being equipped with a stirrer, a heating element, a distillation column and a $CO_2$ trap and being suited for use at pressures of between 6.65 kPa (50 mmHg) and 2 MPa (20 atm). The mixture was heated to a temperature of 280° C and was maintained at this temperature for 25 minutes. After cooling and filtration 700 g of PFPE having a mean molecular weight (MW) of 1500 a.m.u. were recovered in the reactor, while 200 g of a product having an MW of 570 a.m.u. were collected in the $CO_2$ trap.

After treatment with alkali and distillation, the 700 g of resultant PFPE, having an MW of 1500 a.m.u., Provided 490 g of a neutral fraction and 210 g of a mixture of mono- and diacids, while the 200 g of product with an MW of 570 a.m.u. were composed of a mixture in which the acid molecule/neutral molecule ratio was 0.33.

EXAMPLE 4

The same process as described in example 3, conducted with rectification under reduced pressure (6.65 to 26.6 kPa= 50 to 200 mm Hg) provided, at a temperature of 270° C for 25 minutes, 800 g of distillate consisting of perfluoropolyether having an MW of 700 a.m.u. and an acid molecule/neutral molecule ratio of 0.45.

EXAMPLE 5

1000 g of the perfluoropolyether utilized in example 3 as a starting product ($\overline{MW}$ = 4000) and 10 g of $AlF_3$ of the same type as used in example 3 were fed to a 1200 ml metal reactor equipped with a stirrer, a heating element and a $CO_2$ trap. The temperature was brought to 300° C and maintained for 60 minutes.

After heating and filtration 500 g of polyether having a molecular weight of 980 a.m.u. were recovered in the reactor. The ratio of acid molecules to neutral molecules was 0.05. 370 g of a perfluoropolyether having a molecular weight of 560 a.m.u. and an acid molecule/neutral molecule ratio of 0.25 were present in the $CO_2$ trap.

A 400 g portion of the product thus obtained with an MW of 980 a.m.u. was transferred to an Ni reactor and subsequently neutralized with elemental $F_2$ at a temperature of 150° C for 18 hours, thus providing neutral PFPE in a yield of 95%.

Completely analogous results were obtained by using $F_2$ and UV radiation at a temperature of 20° C for 5 hours with the remaining 100 g.

EXAMPLE 6

1000 g of the PFPE utilized in example 3, along with 10 g of a mixture of $CoCl_2$, $FeCl_2$ and $CrCl_3$ in a weight ratio of 1:3:1, were fed to a 1200 ml Ni reactor equipped with a stirrer, heating elements and a $CO_2$ trap.

After the temperature had been brought to 300° C in a nitrogen flow of 10 l/h, $N_2$ was gradually replaced by $F_2$. After a reaction of 10 hours at 120° C, a PFPE sample exhibited a MW of 3500 while, after a further 10 hours, the MW was equal to 2900. At the end of the reaction, 800 g of PFPE having a MW of 2900 and a residual acidity corresponding to 1 acid molecule per 1200 neutral molecules were collected. The resulting product was filtered and then brought to complete conversion of the acid end groups with $F_2$ in a Ni reactor at a temperature of 180° C.

EXAMPLE 7

5 g of $AlF_3$ of the same type as described in example 3 were added to 500 g of perfluoropolyether (PFPE) from $C_3F_6$ and $C_2F_4$, having the general formula (III) and a viscosity of 1200 mm²/s (cSt),in a reactor equipped with a sti rrer, heating elements and a $CO_2$ trap. After a reaction time of 20 minutes at a temperature of 300° C, 380 g of an oil having a viscosity of 45 mm²/s (cSt) were collected after $AlF_3$ had

been filtered off.

50 g of PFPE having a viscosity of 3 mm²/s (cSt) were recovered in the dry ice trap in which the volatile reaction products had condensed.

Distillation under vacuum with rectification of the acid product having a viscosity of 45 mm²/s (cSt) provided 156 g of a head product with a viscosity of 7 mm²/s (cSt). This was placed into a cylindrical photochemical reactor of 150 ml volume and was irradiated for 18 hours with a Hanau lamp TQ 150 which emitted radiation in the range of 250 to 300 nm with a flow of 1.5 . $10^{-3}$ Einstein/minute.

After irradiation, 143 g of an oil having a viscosity of 10 mm²/s (cSt) and a small residue of acid molecules was obtained. Complete neutralization occured with KOH at 220 °C in an autoclave according to the method described in GB-A-1 104 482. 130 g of a neutral oil having a viscosity of 14 mm²/s (cSt) were collected.

EXAMPLE 8

1000 g of PFPE of type (III), utilized in example 7, and 10 g of a mixture of $CoCl_2$, $FeCl_2$ and $CrCl_3$ in a ratio of 1:3:1 were fed to a 1200 ml Ni reactor equipped with a sti rrer, heating elements and a $CO_2$ trap.

After the temperature had been brought to 220 °C in an $N_2$ flow of 10 l/h, $N_2$ was gradually replaced by elemental fluorine. After a reaction time of 11 hours at 220 °C, a PFPE sample exhibited a viscosity of 200 mm²/s (cSt) while, after a further 12 hours, the viscosity was 50 mm²/s (cSt).

When the reaction was complete, 850 g of PFPE, having a viscosity of 50 mm²/s (cSt) and a residual acidity of 0.03 meq/g were collected. The product thus obtained was filtered and then completely neutralized by fluorination of the acid end groups with elemental fluorine in a nickel reactor at a temperature of 180 °C.

EXAMPLE 9

420 g of a mixture of acids prepared according to the process of example 3 and having an osmometrically determined molecular weight of 1500 a.m.u. were esterified with methanol, maintaining a ref lux for 5 hours. After separation of the alcoholic phase the layer consisting of methyl esters of the perfluorinated acids was purified by vacuum evaporation (0.013 kPa = 0.1 Torr) at 100 °C for 2 hours.

425 g of methyl esters were obtained which were subsequently reduced to a mixture of mono- and difunctional alcohols with $NaBH_4$, as described in US-A-3 814 741. By titration with acetic anhydride this alcohol mixture (product B) showed a functionality corresponding to 1.33 alcoholic groups.

50 g of the product were dissolved in a mixture of $CF_2Cl-CCl_2F$ (Freon®113)/ethyl ether and 3.5 g of acryloyl chloride were added in the presence of the stoichiometric molar amount of pyridine. After filtration of pyridine chloride and washing with ice water the corresponding mixture of acrylates of the perfluoropolyether alcohols was obtained in quantitative yield.

A solution of 5% by weight of the acrylate mixture in Freon® 113 was utilized for impregnating a magnetic video tape which, after removal of the solvent, was subjected to electron beam radiation of an intensity of 3 Mrad at a rate of 30 m/minute.

A tape coated with a protective film was obtained on which the angle of contact with water was determined as being 120 °. The corresponding angle for a non-treated tape was 50 °.

EXAMPLE 10

100 g of product B of example 9, dissolved in Freon® 113 and treated with the stoichiometric molar amount of $NaBH_4$, were subsequently reacted with 8 g of ethylene oxide at 50 °C. Upon completion of the react ion and after washing with water and subsequent drying, the mixture provided a product (product D) which exhibited the structure: $R_FO(CF_2-CF_2-CH_2OCH_2CH_2OH)_n$, wherein $R_F$ is the perfluoroether chain described in example 3 and n is 1 or 2.

50 g of product D were added to 17 g of product

$$R'_FO(CF_2CH_2O-CH_2-\underset{OH}{CH}-\underset{OH}{CH_2})_2 \qquad \text{(product E)}$$

wherein $R'_F$ had a structure of the type of Fomblin Z® and an MW of 1500 a.m.u. (product E), prepared

according to US-A-3 972 856 (compound VII).

50 g of the resulting mixture (product F) were reacted with 5.8 g of toluene diisocyanate at 50° C for 3 hours. A polymer having rubber-type characteristics and a glass transition temperature of lower than -80° C was obtained. From this polymer a test piece was prepared which, when subjected to tensile stress, exhibited a tensile strength of 196 $N/cm^2$ (20 $kg/cm^2$) with an elongation of 450%.

A mixture consisting of 17 g of product D and 17 g of product E was reacted with 5.3 g of toluene diisocyanate and 0.1% by weight of a catalyst based on iron acetylacetonate at a temperature of 30° C for 1 hour, whereafter the whole mixture was placed between the plates of a press at 100° C for 3 hours. A sheet exhibiting a tensile strength of 490.5 $N/cm^2$ (50 $kg/cm^2$) and an elongation of 350% was obtained.

EXAMPLE 11

A mixture of 200 g of mono- and diacids, prepared as described in example 3 (MW = 1500), dissolved in Freon® 113, was reacted with 47 g of toluene diisocyanate at 120° C for 2 hours.

After evolution of $CO_2$ and subsequent cooling the excess toluene diisocyanate was washed out with toluene. After evaporation and subsequent drying at 50° C under a vacuum of $1.3 \times 10^{-4}$ kPa ($10^{-3}$ mmHg) a product was obtained with a structure analyzed to be:

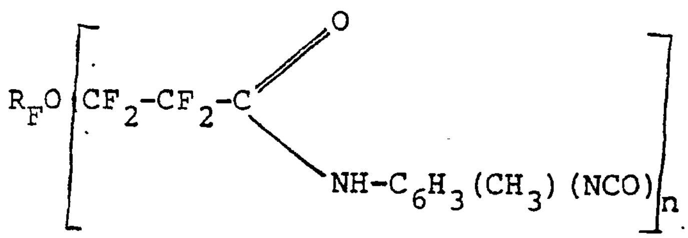

wherein n is 1 or 2 and $R_F$ is the perf luoropolyether chain described in example 3. The presence of isocyanate groups was also confirmed by titration with butylamine.

20 g of this product and 0.05 g of potassium acetate were dissolved to obtain a mixture which was utilized for spreading a film having a thickness of 10 nm (100 Å) onto a rigid magnetic disc, type Winchester, which was placed into an oven at a temperature of 100° C and left there for 10 hours.

A polymer film endowed with excellent wear-resistance and exhibiting a static friction coefficient of 0.2 was obtained on the disc surface.

EXAMPLE 12

9.1 g of phenyl-bis-1,3-(hexafluoropropylidene) alcohol, 10 g of epichlorohydrin and 7 g of NaOH were added to 50 g of ethoxylated alcohols (product D of example 10), dissolved in a solvent mixture consisting of Freon® 113, acetone and t-butanol in a volume ratio of 1:1:1. The reagent mixture was kept at reflux for 8 hours and a very viscous product was obtained which, analyzed by means of a rotary viscosimeter, exhibited a viscosity of 400 Pa.s (4000 poises) at 50° C.

A 50 g portion of the epoxy polymer thus obtained was mixed with 1 g of ethylenediamine and placed between two plates of a press at 60° C, where it was kept for 3 hours.

A rubber-like sheet was obtained from which a test piece was prepared which, under tensile stress, exhibited a tensile strength of 490.5 $N/cm^2$ (50 $kg/cm^2$) and an elongation of 350%.

**Claims**

**Claims for the following Contracting States : AT, CH, ES, IT, LI**

1. A process for cracking perfluoropolyethers of the following classes:

$$\text{I)}\quad R_fO(CF_2CF_2O)_nR_f$$

$$\text{II)}\quad A(CF_2CF_2CF_2O)_nB$$

$$\text{III)}\quad E(CF_2CF_2O)_m(C_3F_6O)_p(CF_2O)_q(\underset{\displaystyle CF_3}{\underset{|}{C}}FO)_rD$$

wherein n, m, p, q and r are integers,
n ranges from 2 to 200; m, p, q and r range from 1 to 100 and the sum m+p+q+r ranges from 4 to 400; $R_f$ is $CF_3$ or $C_2F_5$; A is F or $OR_f$; B and D are perfluoroalkyl groups having 1 to 3 carbon atoms; and E is F or $OR'_f$, where $R'_f$ is a perfluoroalkyl group having 1 to 3 carbon atoms;
which comprises heating the perfluoropolyethers of formulae I, II or III to temperatures ranging from 150 to 380°C in the presence of from 0.1 to 10% by weight, based on the weight of the starting perfluoropolyether, of a catalyst composed of fluorides, oxyfluorides or oxides of transition metals selected from Ti, V, Cr, Mn, Fe, Co, Ni, Cu, Zr, Mo, Zn or of Al, Sn, Sb.

**2.** A process according to claim 1, wherein the cracking is carried out in the presence of fluorine.

**3.** A process according to any one of claims 1 or 2, wherein the crack products are continuously removed from the reacting mixture by distillation.

**4.** Neutral perfluoropolyethers having a low molecular weight, prepared according to the process of claims 1 or 2.

**5.** Perfluoropolyethers obtainable by the process according to claims 1 and 2 consisting of the repeating units:
I') $-CF_2CF_2O-$,
II') $-CF_2CF_2CF_2O-$
wherein both end groups are of the formula:
$-(O)CF_2COF$ for class I') and
$-(O)CF_2CF_2COF$ for class II').

**6.** Perfluoropolyethers according to claim 5 consisting of the repeating units:
I') $-CF_2CF_2O-$,
II') $-CF_2CF_2CF_2O-$
wherein both end groups are of the formula:
$-(O)CF_2COOH$ for class I') and
$-(O)CF_2CF_2COOH$ for class II') obtainable by acidification of the COF end groups with strong acids, and derivatives thereof obtainable by conversion of the carboxylic group to amide and ester groups or the conversion of the latter groups to nitrile, isocyanate and hydroxyl groups.

**Claims for the following Contracting States : BE, DE, FR, GB, NL, SE**

**1.** A process for cracking perfluoropolyethers of the following classes:

$$\text{I)}\quad R_fO(CF_2CF_2O)_nR_f$$

$$\text{II)}\quad A(CF_2CF_2CF_2O)_nB$$

$$\text{III)}\quad E(CF_2CF_2O)_m(C_3F_6O)_p(CF_2O)_q(\underset{\displaystyle CF_3}{\underset{|}{C}}FO)_rD$$

wherein n, m, p, q and r are integers,
n ranges from 2 to 200; m, p, q and r range from 1 to 100 and the sum m+p+q+r ranges from 4 to 400; $R_f$ is $CF_3$ or $C_2F_5$; A is F or $OR_f$; B and D are perfluoroalkyl groups having 1 to 3 carbon atoms; and E is F or $OR'_f$, where $R'_f$ is a perfluoroalkyl group having 1 to 3 carbon atoms;

which comprises heating the perfluoropolyethers of formulae I, II or III to temperatures ranging from 150 to 380 °C in the presence of from 0.1 to 10% by weight, based on the weight of the starting perfluoropolyether, of a catalyst composed of fluorides, oxyfluorides or oxides of transition metals selected from Ti, V, Cr, Mn, Fe, Co, Ni, Cu, Zr, Mo, Zn or of Al, Sn, Sb, provided that in the case of perfluoropolyethers of formula II the catalyst does not comprise fluorides and/or oxyfluorides of Ti, V, Co, Ni or Al or mixtures thereof in amounts of from 0.1 to 2% by weight.

**2.** A process according to claim 1, wherein the cracking is carried out in the presence of fluorine.

**3.** A process according to any one of claims 1 or 2, wherein the crack products are continuously removed from the reacting mixture by distillation.

**4.** Perfluoropolyethers obtainable by the process according to claims 1 and 2, consisting of the repeating units:
I') $-CF_2CF_2O-$,
II') $-CF_2CF_2CF_2O-$
wherein both end groups are of the formula:
$-(O)CF_2COF$ for class I') and
$-(O)CF_2CF_2COF$ for class II'):

**5.** Perfluoropolyethers according to claim 4 consisting of the repeating units:
I') $-CF_2CF_2O-$,
II') $-CF_2CF_2CF_2O-$
wherein both end groups are of the formula:
$-(O)CF_2COOH$ for class I') and
$-(O)CF_2CF_2COOH$ for class II') obtainable by acidification of the COF end groups with strong acids and derivatives thereof obtainable by conversion of the carboxylic group to amide and ester groups or the conversion of the latter groups to nitrile, isocyanate and hydroxyl groups.

**Revendications**

**Revendications pour les Etats contractants suivants : AT, CH, ES, IT, LI**

**1.** Un procédé de craquage de perfluoropolyéthers de formules suivantes:

I) $R_fO(CF_2CF_2O)_nR_f$

II) $A(CF_2CF_2CF_2O)_nB$

III) $E(CF_2CF_2O)_m(C_3F_6O)_p(CF_2O)_q(\underset{CF_3}{CFO})_rD$

dans lesquelles:

- n, m, p, q et r sont des nombres entiers, n varie de 2 à 200; m, p, q et r varient de 1 à 100 et la somme m + p + q + r varie de 4 à 400;
- $R_f$ est $CF_3$ ou $C_2F_5$;
- A est F ou $OR_f$;
- B et D sont des groupes perfluoroalkyles ayant de 1 à 3 atomes de carbone; et
- E est F ou $OR'_f$, dans lequel $R'_f$ est un groupe perfluoroalkyle ayant de 1 à 3 atomes de carbone;

qui comprend le chauffage des perfluoropolyéthers de formules I, II ou III jusqu'à des températures de 150 à 380 °C en présence de 0,1 à 10% en poids, par rapport au poids du perfluoropolyéther de départ, d'un catalyseur composé de fluorures, oxyfluorures ou oxydes des métaux de transition choisis parmi Ti, V, Cr, Mn, Fe, Co, Ni, Cu, Zr, Mo, Zn ou parmi Al, Sn, Sb.

**2.** Un procédé selon la revendication 1, dans lequel le craquage est mis en oeuvre en présence de fluor.

**3.** Un procédé selon l'une quelconque des revendications 1 ou 2, dans lequel les produits du craquage

sont retirés en continu du mélange réactionnel par distillation.

4. Perfluoropolyéthers neutres ayant un poids moléculaire faible, préparés selon le procédé des revendications 1 ou 2.

5. Perfluoropolyéthers obtenus par le procédé selon les revendications 1 et 2, formés des unités répétitives:
I') $-CF_2CF_2O-$
II') $-CF_2CF_2CF_2O-$
dans lesquelles les deux groupes terminaux présentent les formules:
$-(O)CF_2COF$ pour la classe I') et
$-(O)CF_2CF_2COF$ pour la classe II').

6. Perfluoropolyéthers selon la revendication 5, formés des unités répétitives:
I') $-CF_2CF_2O-$
II') $-CF_2CF_2CF_2O-$
dans lesquelles les deux groupes terminaux présentent les formules:
$-(O)CF_2COOH$ pour la classe I') et
$-(O)CF_2CF_2COOH$ pour la classe II'),
obtenus par acidification des groupes terminaux COF avec des acides forts,
et leurs dérivés obtenus par conversion du groupe carboxylique en groupes amide ou ester ou la conversion de ces derniers groupes en des groupes nitrile, isocyanate ou hydroxyle.

**Revendications pour les Etats contractants suivants : BE, DE, FR, GB, NL, SE**

1. Un procédé de craquage de perfluoropolyéthers de formules suivantes:

I) $R_fO(CF_2CF_2O)_nR_f$

II) $A(CF_2CF_2CF_2O)_nB$

III) $E(CF_2CF_2O)_m(C_3F_6O)_p(CF_2O)_q(\underset{CF_3}{CFO})_rD$

dans lesquelles:

n, m, p, q et T sont des nombres entiers, n varie de 2 à 200; m, p, q et r varient de 1 à 100 et la somme m + p + q + r varie de 4 à 400;
$R_f$ est $CF_3$ ou $C_2F_5$;
A est F ou $OR_f$;
B et D sont des groupes perfluoroalkyles ayant de 1 à 3 atomes de carbone; et
E est F ou $OR'_f$, dans lequel $R'_f$ est un groupe perfluoroalkyle ayant de 1 à 3 atomes de carbone;

qui comprend le chauffage des perfluoropolyéthers de formules I, II ou III jusqu'à des températures de 150 à 380 °C en présence de 0,1 à 10% en poids, par rapport au poids du perfluoropolyéther de départ, d'un catalyseur composé de fluorures, oxyfluorures ou oxydes des métaux de transition choisis parmi Ti, V, Cr, Mn, Fe, Co, Ni, Cu, Zr, Mo, Zn ou parmi Al, Sn, Sb, pourvu que, dans le cas des perfluoropolyéthers de formule II, le catalyseur ne comprenne pas de 0,1 à 2% en poids de fluorures et/ou oxyfluorures de Ti, V, Co, Ni ou Al ou des mélanges de ceux-ci.

2. Un procédé selon la revendication 1, dans lequel le craquage est mis en oeuvre en présence de fluor.

3. Un procédé selon l'une quelconque des revendications 1 ou 2, dans lequel les produits du craquage sont retirés en continu du mélange réactionnel par distillation.

4. Perfluoropolyéthers obtenus par le procédé selon les revendications 1 et 2, formés des unités répétitives:
I') $-CF_2CF_2O-$

II') $-CF_2CF_2CF_2O-$
dans lesquelles les deux groupes terminaux présentent les formules:
$-(O)CF_2COF$ pour la classe I') et
$-(O)CF_2CF_2COF$ pour la classe II').

5. Perfluoropolyéthers selon la revendication 4, formés des unités répétitives:
I') $-CF_2CF_2O-$
II') $-CF_2CF_2CF_2O-$
dans lesquelles les deux groupes terminaux présentent les formules:
$-(O)CF_2COOH$ pour la classe I') et
$-(O)CF_2CF_2COOH$ pour la classe II'),
obtenus par acidification des groupes terminaux COF avec des acides forts,
et leurs dérivés obtenus par conversion du groupe carboxylique en groupes amide ou ester ou la conversion de ces derniers groupes en des groupes nitrile, isocyanate ou hydroxyle.

**Patentansprüche**
**Patentansprüche für folgende Vertragsstaaten : AT, CH, ES, IT, LI**

1. Verfahren zum Cracken von Perfluorpolyethern der folgenden Klassen:

$$\text{I)}\ R_fO(CF_2CF_2O)_nR_f$$
$$\text{II)}\ A(CF_2CF_2CF_2O)_nB$$
$$\text{III)}\ E(CF_2CF_2O)_m(C_3F_6O)_p(\underset{\displaystyle CF_3}{\underset{|}{CF_2}}O)_q(CFO)_rD$$

worin n, m, p, q und r ganze Zahlen sind, n im Bereich von 2 bis 200 liegt; m, p, q und r im Bereich von 1 bis 100 liegen und die Summe $m+p+q+r$ in Bereich von 4 bis 400 liegt; $R_f$ $CF_3$ oder $C_2F_5$ ist; A F oder $OR_f$ ist; B und D Perfluoralkylgruppen mit 1 bis 3 Kohlenstoffatomen sind; und E F oder $OR'_f$ ist, worin $R'_f$ eine Perfluoralkylgruppe mit 1 bis 3 Kohlenstoffatomen ist, welches umfaßt das Erhitzen der Perfluorpolyether der Formeln I, II oder III auf Temperaturen von 150 bis 380 °C in Anwesenheit von 0,1 bis 10 Gewichtsprozent, bezogen auf das Gewicht des eingesetzten Perfluorpolyethers, eines Katalysators, der aus Fluoriden, Oxyfluoriden oder Oxiden von Übergangsmetallen, ausgewählt aus Ti, V, Cr, Mn, Fe, Co, Ni, Cu, Zr, Mo, Zn, oder von Al, Sn, Sb zusammengesetzt ist.

2. Verfahren nach Anspruch 1, worin das Cracken in Gegenwart von Fluor durchgeführt wird.

3. Verfahren nach irgendeinem der Ansprüche 1 oder 2, worin die Crackprodukte aus dem Reaktionsgemisch kontinuierlich durch Destillation entfernt werden.

4. Neutrale Perfluorpolyether mit niedrigem Molekulargewicht, hergestellt nach dem Verfahren der Ansprüche 1 oder 2.

5. Perfluorpolyether, erhältlich nach dem Verfahren der Ansprüche 1 und 2 und bestehend aus den Struktureinheiten:
I') $-CF_2CF_2O-$,
II') $-CF_2CF_2CF_2O-$
worin beide Endgruppen die Formel:
$-(O)CF_2COF$ für Klasse I') und
$-(O)CF_2CF_2COF$ für Klasse II') haben.

6. Perfluorpolyether nach Anspruch 5 und bestehend aus den Struktureinheiten:
I') $-CF_2CF_2O-$,
II') $-CF_2CF_2CF_2O-$
worin beide Endgruppen die Formel:
$-(O)CF_2COOH$ für Klasse I') und

-(O)$CF_2CF_2COOH$ für Klasse II') haben,
erhältlich durch Ansäuern der COF-Endgruppen mit starken Säuren,
und Derivate davon, erhältlich durch Überführen der Carbonsäuregruppe in Amid- und Estergruppen oder durch Überführen der letzteren Gruppen in Nitril-, Isocyanat- und Hydroxylgruppen.

**Patentansprüche für folgende Vertragsstaaten : BE, DE, FR, GB, NL, SE**

1. Verfahren zum Cracken von Perfluorpolyethern der folgenden Klassen:

$$\text{I)}\quad R_fO(CF_2CF_2O)_nR_f$$
$$\text{II)}\quad A(CF_2CF_2CF_2O)_nB$$
$$\text{III)}\quad E(CF_2CF_2O)_m(C_3F_6O)_p(CF_2O)_q(\underset{\substack{|\\ CF_3}}{CFO})_rD$$

worin n, m, p, q und r ganze Zahlen sind, n im Bereich von 2 bis 200 liegt; m, p, q und r im Bereich von 1 bis 100 liegen und die Summe m+p+q+r im Bereich von 4 bis 400 liegt; $R_f$ $CF_3$ oder $C_2F_5$ ist; A F oder $OR_f$ ist; B und D Perfluoralkylgruppen mit 1 bis 3 Kohlenstoffatomen sind; und E F oder $OR'_f$ ist, worin $R'_f$ eine perfluoralkylgruppe mit 1 bis 3 Kohlenstoffatomen ist, welches umfaßt das Erhitzen der Perfluorpolyether der Formeln I, II oder III auf Temperaturen von 150 bis 380 °C in Anwesenheit von 0,1 bis 10 Gewichtsprozent, bezogen auf das Gewicht des eingesetzten Perfluorpolyethers, eines Katalysators, der zusammengesetzt ist aus Fluoriden, Oxyfluoriden oder Oxiden von Übergangsmetallen, ausgewählt aus Ti, V, Cr, Mn, Fe, Co, Ni, Cu, Zr, Mo, Zn, oder von Al, Sn, Sb, mit der Maßgabe, daß im Falle von Perfluorpolyethern der Formel II der Katalysator keine Fluoride und/oder Oxyfluoride von Ti, V, Co, Ni oder Al oder Mischungen davon in Mengen von 0,1 bis 2 Gewichtsprozent umfaßt.

2. Verfahren nach Anspruch 1, worin das Cracken in Gegenwart von Fluor durchgeführt wird.

3. Verfahren nach irgendeinem der Ansprüche 1 oder 2, worin die Crackprodukte aus dem Reaktionsgemisch kontinuierlich durch Destillation entfernt werden.

4. Perfluorpolyether, erhältlich nach dem Verfahren der Ansprüche 1 und 2 und bestehend aus den Struktureinheiten:
   I') -$CF_2CF_2O$-,
   II') -$CF_2CF_2CF_2O$-
   worin beide Endgruppen die Formel:
   -(O)$CF_2COF$ für Klasse I') und
   -(O)$CF_2CF_2COF$ für Klasse II') haben.

5. Perfluorpolyether nach Anspruch 4 und bestehend aus den Struktureinheiten:
   I') -$CF_2CF_2O$-,
   II') -$CF_2CF_2CF_2O$-
   worin beide Endgruppen die Formel:
   -(O)$CF_2COOH$ für Klasse I') und
   -(O)$CF_2CF_2COOH$ für Klasse II') haben,
   erhältlich durch Ansäuern der COF-Endgruppen mit starken Säuren,
   und Derivate davon, erhältlich durch Überführen der Carbonsäuregruppe in Amid- und Estergruppen oder durch Überführen der letzteren Gruppen in Nitril-, Isocyanat- und Hydroxylgruppen.